# EUROPÄISCHE PATENTSCHRIFT

(11) **EP 3 386 369 B1**
(45) Veröffentlichungstag und Bekanntmachung des Hinweises auf die Patenterteilung: **30.09.2020**
(21) Anmeldenummer: 16805726.3
(22) Anmeldetag: 01.12.2016
(51) Int. Cl.: A61B 1/00, G02B 23/24, A61B 1/018, A61B 1/06, A61B 17/00

(54) **INSTRUMENT UND VERWENDUNG EINES INSTRUMENTS**
INSTRUMENT AND USE OF AN INSTRUMENT
INSTRUMENT ET UTILISATION D'UN INSTRUMENT

(30) Priorität: 08.12.2015 DE 102015015772
(43) Veröffentlichungstag der Anmeldung: 17.10.2018
(73) Patentinhaber: Schölly Fiberoptic GmbH, 79211 Denzlingen (DE)
(72) Erfinder: FRANZ, Marcus, 79189 Bad Krozingen-Biengen (DE); KRATSCHMER, Matthias, 79114 Freiburg (DE)
(74) Vertreter: Mertzlufft-Paufler, Cornelius
(86) Internationale Anmeldenummer: PCT/EP2016/002031
(87) Internationale Veröffentlichungsnummer: WO 2017/097405

(56) Entgegenhaltungen:
- JP-A- 2014 128 465
- US-A1- 2005 015 113
- US-A1- 2011 152 610
- US-A1- 2015 031 947
- US-A1- 2015 119 918
- US-A1- 2015 257 629

## Beschreibung

Die Offenbarung betrifft ein Instrument mit einem flexiblen Schaft, der ein distales Ende und ein proximales Ende aufweist, wobei an dem proximalen Ende eine Handhabe angeordnet ist und an dem distalen Ende ein Hauptteil ausgebildet ist, wobei ein Ausklappteil über ein Gestänge zwischen einer an dem Hauptteil angeordneten, ersten Position und einer von dem Hauptteil in Bezug auf eine Verlaufsrichtung des Schafts seitlich beabstandeten, zweiten Position verstellbar angeordnet ist.

Die Offenbarung betrifft weiter eine Verwendung eines derartigen Instruments zu einer Bearbeitung, insbesondere einer Reparatur, eines innerhalb eines Hohlraums angeordneten Bauteils.

Hierbei ist es üblich geworden, das erwähnte Gestänge über ein flexibles Zugelement vom proximalen Ende des flexiblen Schafts her zu betätigen. Es ist so ein Umstellen zwischen einer vollständig eingeklappten und einer vollständig ausgeklappten Position des Ausklappteils erreichbar.

Aus medizinischen Anwendungen sind ferner eine Reihe von vergleichbaren Mechaniken bekannt. So offenbaren US 2011/0152610 A1 und JP 2014 128465 A jeweils Endoskope mit einem Ausklapp- bzw. Verschwenkteil, das in eine seitlich von einem Hauptteil des jeweiligen Endoskops beabstandete Position verstellt werden kann.

Aus den Druckschriften US 2015/257629 A1, US 2005/015113 A1, sowie US 2015/119918 A1 sind ferner weitere manuelle Instrumente mit Mechaniken bekannt, die gelenkig miteinander verbundene Gestänge aufweisen, mit denen Schub- oder Zugbewegungen in Verschenk bzw. Ausstellbewegungen umsetzbar sind.

Der Erfindung liegt die Aufgabe zugrunde, die Gebrauchseigenschaften eines Instruments der eingangs beschriebenen Art zu verbessern.

Zur Lösung dieser Aufgabe sind erfindungsgemäß bei einem Instrument die Merkmale des Anspruchs 1 vorgesehen. Insbesondere wird somit bei einem Instrument der eingangs beschriebenen Art zur Lösung der Aufgabe erfindungsgemäß vorgeschlagen, dass ein Spindelantrieb ausgebildet ist, mit welchem das Gestänge betätigbar ist, und dass ein Gewindeteil des Spindelantriebs über eine längs des Schafts geführte flexible Welle antreibbar ist. Die Erfindung stellt somit als eine Alternative zu der bekannten Ansteuerung über Bowdenzüge oder dergleichen Zugelemente eine Betätigung des Gestänges vom proximalen Ende des flexiblen Schafts her über eine flexible Welle und einen Spindelantrieb bereit. Dies hat den Vorteil, dass Beeinträchtigungen der Betätigbarkeit bei stark gekrümmtem Verlauf des flexiblen Schafts reduzierbar oder sogar ganz vermeidbar sind. Mit der Erfindung ist es außerdem möglich, Zwischenstellungen zwischen einer vollständig eingeklappten, ersten Position und einer vollständig ausgeklappten, zweiten Position definiert anzusteuern. Bevorzugt ist das Gewindeteil eine Spindel des Spindelantriebs, die in einer an dem Gestänge angeordneten Spindelmutter läuft.

Bei einer Ausgestaltung der Offenbarung kann vorgesehen sein, dass die flexible Welle in einem Führungskanal in dem flexiblen Schaft angeordnet ist. Somit ist eine Beeinträchtigung der Drehbarkeit der flexiblen Welle zur Ansteuerung des Spindelantriebs auch in eng oder verwinkelt laufenden Zugängen eines Hohlraumes vermeidbar. Besonders günstig ist es dabei, wenn ein lichter Innendurchmesser des Führungskanals derart größer als ein Außendurchmesser der flexiblen Welle gewählt ist, dass die flexible Welle allseitig beabstandet in dem Führungskanal anordenbar ist. Dies kann beispielsweise dadurch erreicht werden, dass der lichte Innendurchmesser des Führungskanals größer als der Außendurchmesser ist.

Bevorzugt ist die flexible Welle als Vollwelle ausgebildet.

Dies ermöglicht eine besonders einfache Fertigung der flexiblen Welle auch bei sehr kleinen Außendurchmessern der Welle.

Bei einer Ausgestaltung der Offenbarung kann vorgesehen sein, dass längs des Schafts ein an dem Ausklappteil endender Arbeitskanal ausgebildet ist. Von Vorteil ist dabei, dass ein Hohlraum, in den das Instrument eingeführt ist, an einer von dem Hauptteil beabstandeten Stelle zugänglich und/oder bearbeitbar ist. Dies kann beispielsweise dafür verwendet werden, um ein zusätzliches Instrument und/oder Verbrauchsmaterial über das Ausklappteil einzuführen.

Besonders günstig ist es hierbei, wenn der Arbeitskanal zumindest in einem zwischen dem Schaft und dem Ausklappteil liegenden Abschnitt flexibel ausgebildet ist. Auf diese Weise kann der Arbeitskanal beim Ausklappen des Ausklappteils ein Mitbewegen seines distalen Endes zulassen. Besonders günstig ist es hierbei, wenn der flexible Abschnitt zug- und/oder stauchfest ausgebildet ist. Von Vorteil ist dabei, dass eine Führung des Ausklappteils während der Betätigung des Gestänges durch den Arbeitskanal erreichbar ist. Dies vereinfacht die konstruktiven Anforderungen an das Gestänge.

Zur Lösung der genannten Aufgabe ist erfindungsgemäß weiterhin vorgesehen, dass das Gestänge einen Kniehebel bildet. Von Vorteil ist dabei, dass eine Betätigungskraft, die durch den Spindelantrieb bereitstellbar ist, über die Hebelwirkung des Kniehebels als eine Ausstellkraft auf das Ausklappteil einbringbar ist, die mit zunehmender Entfernung des Ausklappteils vom Hauptteil zunimmt. Dies ermöglicht ein besonders einfaches Ausstellen des Ausklappteils.

Hierbei ist erfindungsgemäß vorgesehen, dass der Spindelantrieb an einem Kniegelenk des Kniehebels angreift. Somit kann die bereits erwähnte Ausstellkraft besonders groß sein, wenn das Gestänge seine ausgeklappte Position und damit das Ausklappteil die zweite Position erreicht.

Alternativ oder zusätzlich kann hierbei vorgesehen sein, dass ein freies Ende des Kniehebels am Hauptteil angreift. Ein weiteres freies Ende des Kniehebels kann am Ausklappteil angreifen. Auf diese Weise ergibt sich ein einfaches Abstützen des Ausklappteils am Hauptteil in der zweiten Position. Hierbei kann vorgesehen sein, dass die freien Enden des Kniehebels am Hauptteil beziehungsweise am Ausklappteil angelenkt sind. Von Vorteil ist dabei, dass eine definierte Lage des Kniehebels erreichbar ist.

Bei einer Ausgestaltung der Offenbarung kann vorgesehen sein, dass ein Gestängearm des Gestänges mit einem ersten Ende am Ausklappteil angreift und mit einem zweiten Ende an dem Hauptteil verschieblich geführt ist. Von Vorteil ist dabei, dass eine seitliche Stabilisierung in Bezug auf eine Verstellrichtung des Ausklappteils erreichbar ist. Dies ist besonders dann günstig, wenn in einem Arbeitskanal, beispielsweise dem bereits erwähnten Arbeitskanal, ein Verbrauchsmaterial, beispielsweise ein Gas, Fluid und/oder Feststoff, angeordnet ist, das aus einer Zuführeinrichtung abgewickelt ist und daher einen gewundenen Verlauf hat, welcher den Arbeitskanal verbiegen will. Die genannte Ausgestaltung ist vorteilhaft kombinierbar mit der Ausbildung des Gestänges als ein Kniehebel. Hier kann der erwähnte Gestängearm zwischen seinem ersten Ende und seinem zweiten Ende ein Kniegelenk, beispielsweise das bereits erwähnte Kniegelenk, des Kniehebels aufweisen. Der Gestängearm kann bei einer Ausgestaltung am Ausklappteil angreifen, indem er an dem Ausklappteil angelenkt ist. Besonders günstig ist es, wenn die verschiebliche Führung des zweiten Endes an dem Hauptteil durch eine seitliche Abstützung, beispielsweise in einer Führungsnut, erreicht ist.

Bei einer Ausgestaltung der Offenbarung kann vorgesehen sein, dass an dem Ausklappteil ein Kopfteil ausgebildet ist, wobei an dem Kopfteil ein Ansteuerungspunkt ausgebildet ist. Das Kopfteil kann hierbei beweglich an dem Ausklappteil angeordnet sein. Von Vorteil ist dabei, dass eine zusätzliche Beweglichkeit des Ausklappteils bereitgestellt ist, die unabhängig betätigbar von dem Spindelantrieb und/oder gekoppelt bewegbar mit dem Spindelantrieb eingerichtet sein kann. Beispielsweise kann der Ansteuerungspunkt mit dem Gestänge verbunden sein, um eine gekoppelte Beweglichkeit des Kopfteils bei Betätigung des Spindelantriebs zu erreichen. Diese gekoppelte Beweglichkeit kann beispielsweise dadurch erreicht sein, dass der Ansteuerungspunkt mit einem Gestängearm eines Kniehebels, beispielsweise des bereits erwähnten Kniehebels, verbunden ist. Bevorzugt ist der Ansteuerungspunkt mit einem Gestängearm des Kniehebels verbunden, der über ein Kniegelenk, beispielsweise das erwähnte Kniegelenk, des Kniehebels mit dem Ausklappteil gelenkig verbunden ist.

Bei einer Ausgestaltung der Offenbarung kann vorgesehen sein, dass eine Verbindung des Ansteuerungspunkts mit dem Gestänge so ausgebildet ist, dass das Kopfteil in der ersten Position des Ausklappteils und in der zweiten Position des Ausklappteils zueinander versetzte Ausrichtungen einnimmt. Von Vorteil ist dabei, dass mit dem Kopfteil während des Ausklappens aus der ersten Position in die zweite Position zusätzlich eine Dreh- oder Schwenkbewegung ausführbar ist, durch welche sich eine relative Ausrichtung des Kopfteils in Bezug auf das Hauptteil ändert. Somit ist ein Einrollen des Kopfteils bei Einklappen des Ausklappteils erreichbar. Der genaue Verlauf dieser bevorzugt mit dem Spindelantrieb gekoppelten Bewegung ist durch die Positionierung der Verbindungspunkte an dem Gestänge und die Länge der jeweiligen Verbindungen vorgebbar. Beispielsweise ist erreichbar, dass das Kopfteil in der ersten Position des Ausklappteils und in der zweiten Position des Ausklappteils zueinander entgegengesetzte Ausrichtungen einnimmt.

Bei einer Ausgestaltung der Offenbarung kann vorgesehen sein, dass das Gestänge eine weitere Verstellvorrichtung aufweist, mit welcher zumindest ein Kopfteil, beispielsweise das bereits erwähnte Kopfteil, des Ausklappteils bei feststehendem Spindelantrieb zumindest in der zweiten Position relativ zu dem Hauptteil verstellbar ist. Von Vorteil ist dabei, dass eine Ausrichtung des Kopfteils bei ausgeklapptem Ausklappteil veränderbar ist.

Bei einer Ausgestaltung der Offenbarung kann vorgesehen sein, dass das Hauptteil als ein starres Teil ausgebildet ist. Von Vorteil ist dabei, dass eine belastbare Basis für eine Abstützung des Ausklappteils während des Ausklappens erreichbar ist. Es ist so auch eine definierte Führung einer Ausklappbewegung erreichbar. Alternativ oder zusätzlich kann vorgesehen sein, dass das Ausklappteil als ein starres Teil ausgebildet ist. Von Vorteil ist dabei, dass ein definierter Angriff des Gestänges an dem Ausklappteil erreichbar ist.

Es ist wünschenswert, wenn die starren Teile des Instruments am distalen Ende eine möglichst geringe Baulänge einnehmen, damit das Instrument möglichst flexibel in einen Hohlraum eingeführt werden kann. Hierzu kann vorgesehen sein, dass das Ausklappteil in der ersten Position in einer Aufnahme des Hauptteils aufgenommen ist. Dies ermöglicht einen möglichst kleinen Außendurchmesser des Instruments am distalen Ende in der ersten Position des Ausklappteils.

Alternativ oder zusätzlich kann vorgesehen sein, dass an dem Hauptteil in einem Bereich, an welchem das Ausklappteil in der ersten Position an dem Hauptteil anliegt, wenigstens eine Arbeits- und/oder Beobachtungseinheit ausgebildet ist. Von Vorteil ist dabei, dass eine Verlängerung des Hauptteils über eine Erstreckung des Ausklappteils hinaus in Verlaufsrichtung nicht erforderlich ist. Beispielsweise kann die Arbeits- und/oder Beobachtungseinheit eine Beleuchtungseinheit und/oder eine Kamera und/oder eine Laserlicht-Austrittsstelle sein. Es ist/sind somit eine Beobachtung über eine Kamera, eine Beleuchtung einer zu bearbeitenden Stelle und/oder eine Bearbeitung einer zu bearbeitenden Stelle mit Laserlicht ausführbar.

Bei einer Ausgestaltung der Offenbarung kann vorgesehen sein, dass eine Zufuhreinrichtung für ein in einem Arbeitskanal, beispielsweise dem bereits erwähnten Arbeitskanal, gefördertes Verbrauchsmaterial ausgebildet ist. Dieses Verbrauchsmaterial kann beispielsweise ein Gas, Fluid und/oder ein Feststoff sein. Von Vorteil ist dabei, dass eine Bearbeitung unter Verwendung eines Verbrauchsmaterials in einem Hohlraum mit dem Instrument einfach durchführbar ist.

Bei einer Ausgestaltung der Offenbarung kann vorgesehen sein, dass an dem Hauptteil eine Bearbeitungseinheit ausgebildet ist. Diese Bearbeitungseinheit kann beispielsweise eine Fräs-, Bohr- und/oder Schneideeinheit sein. Von Vorteil ist dabei, dass eine Beobachtung der Verwendung der Bearbeitungseinheit mittels des Ausklappteils erreichbar ist, wenn an dem Ausklappteil eine entsprechende Arbeits- und/oder Beobachtungseinheit ausgebildet ist. Umgekehrt kann die Bearbeitungseinheit, insbesondere die Fräs-, Bohr- und/oder Schneideeinheit, an dem Ausklappteil ausgebildet sein. In diesem Fall ist eine Begutachtung der Bearbeitungseinheit durch eine Arbeits- und/oder Beobachtungseinheit an dem Hauptteil erreichbar.

Bei einer Ausgestaltung der Offenbarung sind an dem Hauptteil und an dem Ausklappteil jeweils Arbeits- und/oder Beobachtungseinheiten ausgebildet, beispielsweise um ein : stereoskopisches Sehen zu ermöglichen.

Bei einer Ausgestaltung der Offenbarung kann vorgesehen sein, dass in dem flexiblen Schaft eine Laserfiber, also beispielsweise eine Lichtleitfaser für Laserlicht, einer Laser-Austrittsstelle an dem Hauptteil und/oder an dem Ausklappteil zugeführt ist. Von Vorteil ist dabei, dass die Ausbildung eines Laser-Generators am distalen Ende verzichtbar ist. Vielmehr kann der Laser-Generator am proximalen Ende ausgebildet sein, wobei das Laserlicht über die Laserfiber eingekoppelt wird und/oder einkoppelbar ist.

Bei einer Ausgestaltung der Offenbarung kann vorgesehen sein, dass an dem Ausklappteil eine Kamera, beispielsweise die bereits erwähnte Kamera, und/oder eine Beleuchtungseinheit, beispielsweise die bereits erwähnte Beleuchtungseinheit, ausgebildet ist/sind. Alternativ oder zusätzlich kann vorgesehen sein, dass an dem Hauptteil eine Kamera, beispielsweise die bereits erwähnte Kamera, und/oder eine Beleuchtungseinheit, beispielsweise die bereits erwähnte Beleuchtungseinheit, ausgebildet ist/sind. Die Offenbarung ermöglicht somit eine unterschiedliche Belegung des Hauptteils und des Ausklappteils mit unterschiedlichen Funktionen, beispielsweise für eine Beleuchtung einer Bearbeitungsstelle aus unterschiedlichen Richtungen und/oder einer Betrachtung einer Bearbeitungsstelle aus unterschiedlichen Richtungen, insbesondere zum stereoskopischen Sehen.

Besonders günstig ist es, wenn das Instrument als ein Visualisierungsinstrument ausgebildet ist. Bevorzugt ist das Instrument als ein Endoskop ausgebildet. Somit sind die bekannten Einsatzgebiete von Visualisierungsinstrumenten und Endoskopen für das offenbarte Instrument erschlossen.

Eine bevorzugte Anwendung der Offenbarung besteht in der Verwendung eines offenbarten Instruments, insbesondere wie zuvor beschrieben und/oder nach einem der auf ein Instrument gerichteten Ansprüche, zu einer Reparatur eines innerhalb eines Hohlraums angeordneten Bauteils. Hierbei ist vorgesehen, dass mit dem Instrument in den Hohlraum ein Verbrauchsmaterial, beispielsweise das bereits erwähnte Verbrauchsmaterial, bevorzugt ein Gas, Fluid und/oder ein Feststoff, insbesondere das bereits erwähnte Gas, das bereits erwähnte Fluid und/oder der bereits erwähnte Feststoff, gefördert wird, mit welchem an einer defekten Stelle des Bauteils eine Ausnehmung ausgefüllt und/oder ein Materialaufbau gebildet wird. Es sind auf diese Weise auch Beschichtungen und/oder Veredelungen von Oberflächen ausführbar. Alternativ oder zusätzlich sind auch Fügeverfahren ausführbar. Somit ist eine Ausbesserung von schwer zugänglichen Bauteilen ermöglicht, ohne dass ein Ausbau des Bauteils erforderlich ist. Beispielsweise kann das Bauteil eine Rohrinnenwand oder ein bewegliches und/oder einem Partikelstrom ausgesetztes Bauteil sein. Die bereits erwähnte Ausnehmung kann beispielsweise mit einer Bearbeitungseinheit des Instruments oder eines weiteren, gleichartigen oder zumindest offenbarten Instruments gefertigt worden sein.

Die in Anspruch 13 definierte erfindungsgemäße Verwendung des Instruments eignet sich besonders gut zur Ausbesserung von Rissen, Oberflächenabplatzungen und/oder gelösten Schichten und dergleichen, wobei die Ausnehmung bevorzugt so eingebracht ist, dass der erwähnte Riss und/oder die abgeplatzte Oberfläche und/oder die gelöste Schicht vollständig beseitigt ist und nicht weiter wandern oder sich ausdehnen kann.

Die Offenbarung wird nun anhand von Ausführungsbeispielen näher beschrieben, ist aber nicht auf diese Ausführungsbeispiele beschränkt. Weitere Ausführungsbeispiele ergeben sich durch Kombination der Merkmale einzelner oder mehrerer Schutzansprüche untereinander und/oder mit einzelnen oder mehreren Merkmalen der Ausführungsbeispiele.

In teilweise stark vereinfachter, schematisierter Darstellung zur Erläuterung der Offenbarung:
- Figur 1: eine Verwendung eines offenbarten Instruments,
- Figur 2: eine Schnittdarstellung durch das distale Ende eines offenbarten Instruments,
- Figur 3: eine Schnittdarstellung mit senkrecht zur Verlaufsrichtung des Instruments verlaufender Schnittebene durch das distalen Ende des Instruments,
- Figur 4: das distale Ende eines offenbarten Instruments mit eingeklapptem Ausklappteil in erster Position,
- Figur 5: das distale Ende aus Figur 4 mit teilweise ausgeklapptem Ausklappteil in einer Zwischenposition,
- Figur 6: das distale Ende gemäß Figur 4 mit vollständig ausgeklapptem Ausklappteil in der zweiten Position,
- Figur 7: das distale Ende eines weiteren offenbarten Instruments mit zusätzlich schwenkbarem Kopfteil,
- Figur 8: das distale Ende gemäß Figur 7 mit gegenüber der Stellung aus Figur 7 versetzter Ausrichtung des Kopfteils und

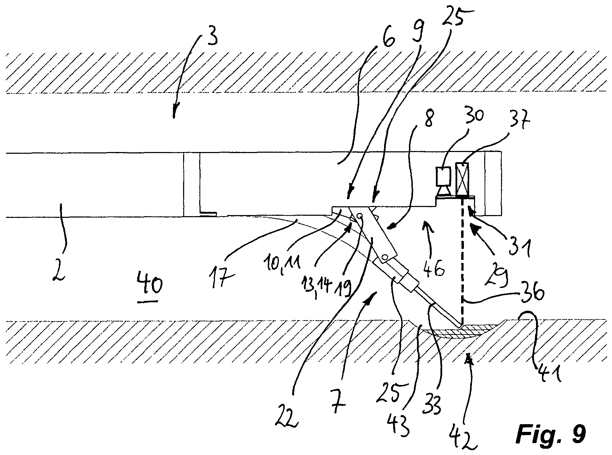

- Figur 9: eine offenbarte Verwendung eines offenbarten Instruments zur Reparatur eines schwer zugänglichen Bauteils.

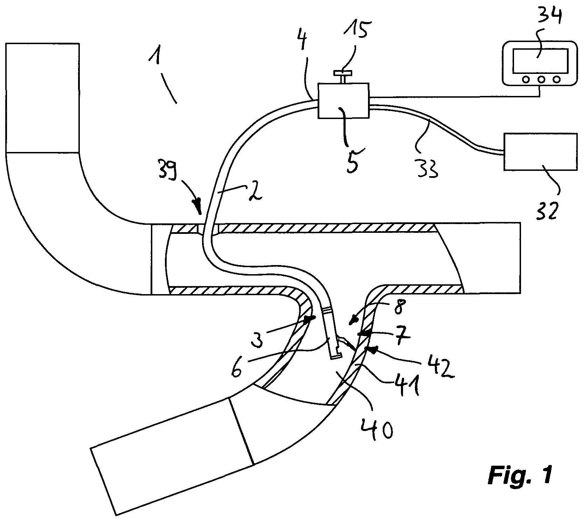

Figur 1 zeigt im Ganzen mit 1 bezeichnetes offenbartes Instrument. Das Instrument 1 hat einen flexiblen Schaft 2, der zwischen einem distalen Ende 3 und einem proximalen Ende 4 verläuft. An dem proximalen Ende 4 ist eine Handhabe 5 ausgebildet, mit welcher der flexible Schaft 2 in an sich bekannter Weise ansteuerbar ist.

An dem distalen Ende 3 ist ein Hauptteil 6 ausgebildet.

Ein Ausklappteil 7 ist über ein Gestänge 8 an dem Hauptteil 6 befestigt und kann zwischen einer ersten Position, in der es an dem Hauptteil 6 anliegt, und einer zweiten Position, in der es von dem Hauptteil 6 ausgestellt und somit beabstandet angeordnet ist, verstellt werden.

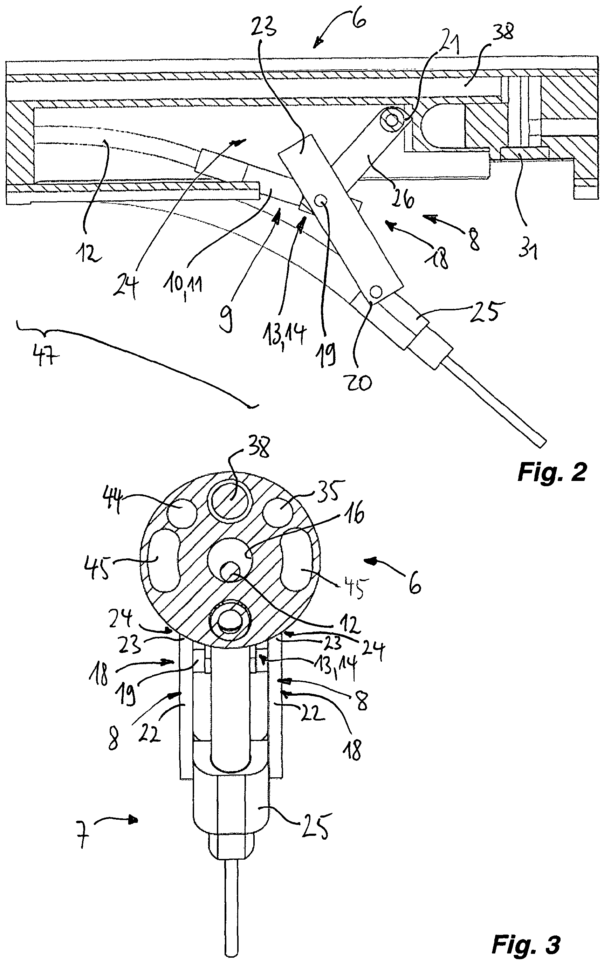

Zur Verstellung des Ausklappteils 7 ist ein Spindelantrieb 9 ausgebildet, der in Figur 2 näher dargestellt ist.

Der Spindelantrieb 9 hat ein Gewindeteil 10, hier eine Spindel 11, das beziehungsweise die mit einer flexiblen Welle 12 drehfest verbunden ist.

Der Spindelantrieb 9 hat ein Gegengewindeteil 13, hier eine Spindelmutter 14, das beziehungsweise die mit dem Gestänge 8 verbunden ist und das Gewindeteil 10 zur Bildung des Spindelantriebs 9 aufnimmt.

Die flexible Welle 12 ist längs des flexiblen Schafts 2 zur Handhabe 5 geführt und über ein Verstellelement 15 an der Handhabe 5 antreibbar. Dieses Verstellelement kann manuell und/oder motorisch betreibbar sein.

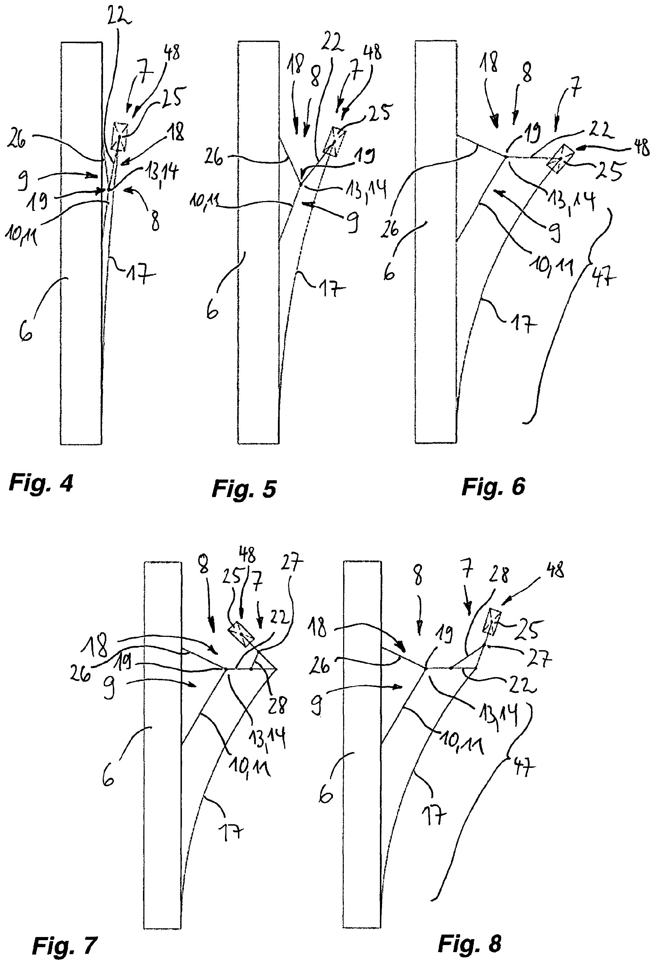

Somit ist der Spindelantrieb 9 durch das Verstellelement 15 vom proximalen Ende 4 des flexiblen Schafts 2 her antreibbar. Hierdurch ist ein Ausstellen des Ausklappteils 7 von einer ersten, eingeklappten Position in eine zweite, ausgeklappte Position erreichbar, wie es die Figuren 4 und 6 beispielhaft darstellen. Es sind auch definierte Zwischenpositionen gemäß Figur 5 einnehmbar. Figur 3 zeigt, dass die flexible Welle 12 in einem Führungskanal 16 angeordnet ist, der entlang des flexiblen Schafts 2 zum proximalen Ende 4 geführt ist. Der lichte Innendurchmesser des Führungskanals 16 ist deutlich größer als der Außendurchmesser der flexiblen Welle 12 gewählt. Dies ermöglicht eine Anordnung der flexiblen Welle 12 in dem Führungskanal 16, in der die flexible Welle 12 abschnittsweise einseitig in dem Führungskanal 16 anliegt. Es ist erkennbar, dass der lichte Innendurchmesser des Führungskanals 16 größer als der Außendurchmesser der flexiblen Welle 12 gewählt ist, um genügend Bewegungsfreiheit zu belassen.

An dem Instrument 1 ist weiter ein Arbeitskanal 17 ausgebildet, der vom proximalen Ende 4 zum distalen Ende 3 verläuft und am Ausklappteil 7 endet. Der Arbeitskanal 17 ist in dem Abschnitt zwischen dem flexiblen Schaft 2 und dem Ausklappteil 7 selbst flexibel, aber zug- und/oder stauchfest ausgebildet. Dies ermöglicht, dass der Arbeitskanal 17 ein seitliches Ausstellen des Ausklappteils 7 nachvollzieht und gleichzeitig das Ausklappteil 7 beim Ausstellen des Gestänges 8 führt.

Das Gestänge 8 bildet einen Kniehebel 18, dessen Kniegelenk 19 mit dem Spindelantrieb 9, genauer mit dem Gegengewindeteil 13, fest verbunden ist. Der Spindelantrieb 9 greift somit an dem Kniegelenk 19 an, um das Gestänge 8 aufzustellen.

Ein erstes freies Ende 20 des Kniehebels 18 ist an dem Ausklappteil angelenkt, während ein zweites freies Ende 21 des Kniehebels 18 an dem Hauptteil angelenkt ist.

Das erste freie Ende 20 ist hierbei an einem ersten Gestängearm 22, der einen Hebelarm des Kniehebels 18 bildet, ausgebildet. Das in Bezug auf das Kniegelenk 19 gegenüberliegende, zweite Ende 23 des ersten Gestängearms 22 ist in einer Führungsnut 24 an dem Hauptteil 6 geführt und seitlich abgestützt.

In Figur 3 ist erkennbar, dass der Kniehebel 18 doppelt ausgeführt ist, wodurch sich eine besonders hohe seitliche Stabilität ergibt. Die doppelte Ausbildung kann in dem gezeigten Beispiel beispielsweise dadurch charakterisiert werden, dass die Kniegelenke 19 eine gemeinsame Achse definieren.

Die Figuren 7 und 8 zeigen eine Variante eines offenbarten Instruments 1, bei welchem das Kopfteil 25 einen Ansteuerungspunkt 27 aufweist, der mit dem Gestänge 8 derart verbunden ist, dass das Kopfteil 25 unterschiedliche Ausrichtungen einnehmen kann.

Hierzu weist das Gestänge 8 eine Verstellvorrichtung 28 auf, mit welcher das Kopfteil 25 unabhängig von dem Spindelantrieb 9 schwenkbar ist.

Bei einer Variante von Figur 7 und 8 ist der Ansteuerungspunkt 27 nicht mit dem ersten Gestängearm 22, sondern mit dem zweiten Gestängearm 26 des Kniehebels verbunden, wodurch ein mit dem Spindelantrieb 9 synchronisiertes Einklappen des Kopfteils 25 erreichbar ist.

Das Hauptteil 6 und das Ausklappteil 7 sind jeweils als starres Teil ausgebildet. An dem Hauptteil 6 ist eine Aufnahme 46 ausgebildet, in welche das Ausklappteil 7 in der ersten Position einlegbar ist.

Eine Arbeits- und/oder Beobachtungseinheit 29 ist in dieser Aufnahme 46 ebenfalls ausgebildet und umfasst eine Kamera 30 und eine Laserlicht-Austrittsstelle 31. Die Kamera 30 kann mit einer integrierten Beleuchtungseinheit ausgebildet sein (nicht dargestellt).

Die Arbeits- und/oder Beobachtungseinheit 29 ist zumindest in der zweiten Position des Ausklappteils 7 aktiviert.

Am proximalen Ende 4 wird dem Arbeitskanal 17 aus einer Zuführeinrichtung 32 Verbrauchsmaterial 33 zugeführt. Dieses Verbrauchsmaterial 33 kann ein Gas, Fluid und/oder Feststoff, beispielsweise ein Schweißdraht, sein.

Ein Monitor 34 ist über einen elektrischen oder optischen Bildleiter 35 an die Arbeits- und/oder Beobachtungseinheit 29, insbesondere an die Kamera 30 angeschlossen, um eine Verarbeitung des Verbrauchsmaterials 33 zu überwachen.

Die Verarbeitung des Verbrauchsmaterials 33 geschieht mit Laserlicht 36, das von einem Lasergenerator 37 im Hauptteil 6 erzeugt oder über eine Laserfiber zugeführt 38 ist und an der Laserlicht-Austrittsstelle 31 austritt.

An dem Hauptteil 6 und/oder an dem Ausklappteil 7 kann in weiteren Ausführungsbeispielen eine Bearbeitungseinheit 48, insbesondere eine Fräs-, Bohr- und/oder Schneideeinheit ausgebildet sein.

Zusätzlich kann an dem Hauptteil 6, insbesondere als Teil der Arbeits- und/oder Beobachtungseinheit 29, eine Beleuchtungseinheit ausgebildet sein.

Durch die Verwendung einer Kamera 30 oder eines optischen Bildleiters 35 ist das Instrument als Visualisierungsinstrument, insbesondere als Endoskop, ausbildbar.

In Figur 3 ist ferner ersichtlich, dass in dem flexiblen Schaft 2 ein Lichtleiter 44 für eine Beleuchtung (nicht dargestellt) der Bearbeitungsstelle ausgebildet ist.

Ferner sind Hohlräume für eine Prozesskomponentenzufuhr, insbesondere eine Prozessgaszufuhr, während der Verarbeitung des Verbrauchsmaterials 33 ausgebildet.

Ist an dem Ausklappteil 7, insbesondere an dem Kopfteil 25, statt oder zusätzlich zu der Bearbeitungseinheit 48 eine Kamera (nicht dargestellt) ausgebildet, kann sogar ein stereoskopisches Betrachten oder Betrachten aus einer Distanz durchgeführt werden. Ist an dem Ausklappteil 7, insbesondere an dem Kopfteil 25, statt oder zusätzlich zu der Bearbeitungseinheit 48 oder der Kamera eine Beleuchtungseinheit (nicht dargestellt) ausgebildet, kann eine Beleuchtung aus einem anderen Winkel - beispielsweise zur Vermeidung von ungünstigen Schattenwürfen - oder eine Beleuchtung mit einer anderen Wellenlänge durchgeführt werden.

Eine offenbarte Verwendung des Instruments 1 sieht vor, dass das Instrument 1 über eine Zugangsöffnung 39 einem Hohlraum 40 zugeführt wird.

In dem Hohlraum 40 ist ein schwer zugängliches Bauteil 41 - hier beispielhaft eine Rohrinnenwand - angeordnet, an welcher eine defekte Stelle 42 vorhanden ist. Diese defekte Stelle 42 wird zu einer Ausnehmung 43 gearbeitet und anschließend mit dem Verbrauchsmaterial 33 ausgefüllt. Es ist auch möglich, ohne Ausarbeitung einer Ausnehmung 43 Verbrauchsmaterial 33 aufzubringen, um einen Materialaufbau auszuführen. Bei der Verarbeitung des Verbrauchsmaterials 33 wird auf das Verbrauchsmaterial 33 mit Laserlicht 36 eingewirkt. Dies kann in Form eines Schweißvorgangs, eines Lötvorgangs oder auch eines Aushärtungsvorgangs für ein Klebematerial sein.

Bei dem Instrument 1 wird vorgeschlagen, am distalen Ende 3 eines flexiblen Schafts 2 ein Hauptteil 6 und ein von diesem ausstellbares Ausklappteil 7 auszubilden, wobei das Ausklappteil 7 über ein von einem Spindelantrieb 9 verstellbares Gestänge 8 ausstellbar ist und wobei der Spindelantrieb 9 von einem proximalen Ende 4 des flexiblen Schafts 2 über eine flexible Welle 12 antreibbar ist.

### Bezugszeichenliste

- 1: Instrument
- 2: flexibler Schaft
- 3: distales Ende
- 4: proximales Ende
- 5: Handhabe
- 6: Hauptteil
- 7: Ausklappteil
- 8: Gestänge
- 9: Spindelantrieb
- 10: Gewindeteil
- 11: Spindel
- 12: flexible Welle
- 13: Gegengewindeteil
- 14: Spindelmutter
- 15: Verstellelement
- 16: Führungskanal
- 17: Arbeitskanal
- 18: Kniehebel
- 19: Kniegelenk
- 20: erstes freies Ende
- 21: zweites freies Ende
- 22: erster Gestängearm
- 23: zweites Ende
- 24: Führungsnut
- 25: Kopfteil
- 26: zweiter Gestängearm
- 27: Ansteuerungspunkt
- 28: Verstellvorrichtung
- 29: Arbeits- und/oder Beobachtungseinheit
- 30: Kamera
- 31: Laserlicht-Austrittsstelle
- 32: Zuführungseinrichtung
- 33: Verbrauchsmaterial
- 34: Monitor
- 35: Bildleiter
- 36: Laserlicht
- 37: Laser-Generator
- 38: Laserfiber
- 39: Zugangsöffnung
- 40: Hohlraum
- 41: Bauteil
- 42: defekte Stelle
- 43: Ausnehmung
- 44: Lichtleiter
- 45: Hohlräume für Schutzgaszufuhr
- 46: Aufnahme
- 47: Abschnitt
- 48: Bearbeitungseinheit

## Patentansprüche

1. Instrument (1) mit einem flexiblen Schaft (2), der ein distales Ende (3) und ein proximales Ende (4) aufweist, wobei an dem proximalen Ende (4) eine Handhabe (5) angeordnet ist und an dem distalen Ende (3) ein Hauptteil (6) ausgebildet ist, wobei ein Ausklappteil (7) über ein Gestänge (8) zwischen einer an dem Hauptteil (6) angeordneten, ersten Position und einer von dem Hauptteil (6) in Bezug auf eine Verlaufsrichtung des Schafts (2) seitlich beabstandeten, zweiten Position verstellbar angeordnet ist, wobei ein Spindelantrieb (9) ausgebildet ist, mit welchem das Gestänge (8) betätigbar ist, wobei ein Gewindeteil (10) des Spindelantriebs (9) über eine längs des Schafts (2) geführte flexible Welle (12) antreibbar ist und wobei der Spindelantrieb (9) ein Gegengewindeteil (13) hat, das mit dem Gestänge (8) verbunden ist und das Gewindeteil (10) zur Bildung des Spindelantriebs (9) aufnimmt, **dadurch gekennzeichnet, dass** das Gestänge (8) einen Kniehebel (18) bildet, wobei der Spindelantrieb (9) an einem Kniegelenk (19) des Kniehebels (18) angreift.

2. Instrument (1) nach Anspruch 1, **dadurch gekennzeichnet, dass** die flexible Welle (12) in einem Führungskanal (16) in dem Schaft (2) angeordnet ist und/oder dass längs des Schafts (2) ein an dem Ausklappteil (7) endender Arbeitskanal (17) ausgebildet ist, insbesondere wobei der Arbeitskanal (17) zumindest in einem zwischen dem Schaft (2) und dem Ausklappteil (7) liegenden Abschnitt (47) flexibel, insbesondere zug- und/oder stauchfest, ausgebildet ist.

3. Instrument (1) nach Anspruch 1 oder 2, **dadurch gekennzeichnet, dass** ein freies Ende (20, 21) des Kniehebels (18) am Hauptteil (6) und/oder am Ausklappteil (7) angreift, insbesondere angelenkt ist.

4. Instrument (1) nach einem der Ansprüche 1 bis 3, **dadurch gekennzeichnet, dass** ein Gestängearm (22, 26) des Gestänges (8) mit einem ersten Ende (20) am Ausklappteil angreift, insbesondere angelenkt ist, und mit einem zweiten Ende (23) an dem Hauptteil (6) verschieblich, insbesondere seitlich abgestützt, geführt ist.

5. Instrument (1) nach einem der Ansprüche 1 bis 4, **dadurch gekennzeichnet, dass** an dem Ausklappteil (7) ein Kopfteil (25) ausgebildet ist, wobei an dem Kopfteil (25) ein vorzugsweise mit dem Gestänge (8) verbundener Ansteuerungspunkt (27) ausgebildet ist, insbesondere wobei der Ansteuerungspunkt (27) mit einem Gestängearm (22, 26) des oder eines Kniehebels (18) verbunden ist.

6. Instrument (1) nach einem der Ansprüche 1 bis 5, **dadurch gekennzeichnet, dass** eine Verbindung des Ansteuerungspunktes (27) mit dem Gestänge (8) so ausgebildet ist, dass das oder ein Kopfteil (25) des Ausklappteils (7) in der ersten Position des Ausklappteils (7) und in der zweiten Position des Ausklappteils (7) zueinander versetzte, insbesondere entgegengesetzte, Ausrichtungen einnimmt.

7. Instrument (1) nach einem der Ansprüche 1 bis 6, **dadurch gekennzeichnet, dass** das Gestänge (8) eine weitere Verstellvorrichtung (28) aufweist, mit welcher zumindest das oder ein Kopfteil (25) des Ausklappteils (7) bei feststehendem Spindelantrieb (9) zumindest in der zweiten Position relativ zu dem Hauptteil (6) verstellbar ist.

8. Instrument (1) nach einem der Ansprüche 1 bis 7, **dadurch gekennzeichnet, dass** das Hauptteil (6) und/oder das Ausklappteil (7) als ein starres Teil ausgebildet ist/sind.

9. Instrument (1) nach einem der Ansprüche 1 bis 8, **dadurch gekennzeichnet, dass** das Ausklappteil (7) in der ersten Position in einer Aufnahme (46) des Hauptteils (6) aufgenommen ist und/oder dass an dem Hauptteil (6) in einem Bereich, an welchem das Ausklappteil (7) in der ersten Position an dem Hauptteil (6) anliegt, wenigstens eine Arbeits- und/oder Beobachtungseinheit (29), insbesondere eine Beleuchtungseinheit, eine Kamera (30) und/oder eine Laserlicht-Austrittsstelle (31), ausgebildet ist.

10. Instrument (1) nach einem der Ansprüche 1 bis 9, **dadurch gekennzeichnet, dass** eine Zufuhreinrichtung (32) für ein in dem oder einem Arbeitskanal (17) gefördertes Verbrauchsmaterial (33), insbesondere für ein Gas, ein Fluid und/oder einen Feststoff, ausgebildet ist und/oder dass an dem Hauptteil (6) und/oder an dem Ausklappteil (7) eine Bearbeitungseinheit, insbesondere eine Fräs-, Bohr- und/oder Schneideinheit, ausgebildet ist.

11. Instrument (1) nach einem der Ansprüche 1 bis 10, **dadurch gekennzeichnet, dass** in dem Schaft (2) eine Laserfiber (38) und/oder einer Laserlicht-Austrittstelle (31) an dem Hauptteil (6) und/oder an dem Ausklappteil (7) zugeführt ist und/oder dass an dem Ausklappteil (7) und/oder dem Hauptteil (6) die oder eine Kamera (30) und/oder die oder eine Beleuchtungseinheit ausgebildet ist/sind.

12. Instrument (1) nach einem der Ansprüche 1 bis 11, **dadurch gekennzeichnet, dass** das Instrument (1) als ein Visualisierungsinstrument, insbesondere als ein Endoskop, ausgebildet ist.

13. Verwendung eines Instruments (1) nach einem der vorangehenden Ansprüche zu einer Bearbeitung, insbesondere einer Reparatur, eines innerhalb eines Hohlraums (40) angeordneten Bauteils (41), insbesondere einer Rohrinnenwand oder ein bewegliches und/oder einem Partikelstrom ausgesetztes Bauteil, wobei mit dem Instrument (1) in den Hohlraum (40) das oder ein Verbrauchsmaterial (33), insbesondere das oder ein Gas, Fluid und/oder Feststoff, gefördert wird, mit welchem an einer defekten Stelle (42) des Bauteils (41) eine Ausnehmung (43) ausgefüllt und/oder ein Materialaufbau gebildet und/oder eine Beschichtung und/oder eine Oberflächenveredelung aufgebracht und/oder ein Fügeverfahren ausgeführt wird.

## Claims

1. Instrument (1) having a flexible stem (2) which has a distal end (3) and a proximal end (4), wherein a handling element (5) is disposed on the proximal end (4), and a main part (6) is configured on the distal end (3), wherein a fold-out part (7) is disposed so as to be adjustable by way of a linkage (8) between a first position, disposed on the main part (6), and a second position which in relation to a profile direction of the stem (2) is laterally spaced apart from the main part (6), wherein a spindle drive (9) by way of which the linkage (8) is activatable is configured, wherein a threaded part (10) of the spindle drive (9) is drivable by way of a flexible shaft (12) that is routed along the stem (2), and wherein the spindle drive (9) has a mating threaded part (13) which is connected to the linkage (8) and receives the threaded part (10) in order for the spindle drive (9) to be formed, **characterized in that** the linkage (8) forms a toggle lever (18), wherein the spindle drive (9) engages on a knuckle joint (19) of the toggle lever (18).

2. Instrument (1) according to Claim 1, **characterized in that** the flexible shaft (12) is disposed in a guide duct (16) in the stem (2), and/or **in that** an operation duct (17) that ends on the fold-out part (7) is configured along the stem (2), in particular wherein the operation duct (17) is configured so as to be flexible at least in a portion (47) that lies between the stem (2) and the fold-out part (7), in particular so as to be tractionresistant and/or compression-resistant.

3. Instrument (1) according to either of Claims 1 and 2, **characterized in that** a free end (20, 21) of the toggle lever (18) engages, in particular is articulated, on the main part (6) and/or on the fold-out part (7).

4. Instrument (1) according to one of Claims 1 to 3, **characterized in that** a linkage arm (22, 26) of the linkage (8) by way of a first end (20) engages, in particular is articulated, on the fold-out part, and by way of a second end (23) is guided so as to be displaceable, in particular laterally supported, on the main part (6).

5. Instrument (1) according to one of Claims 1 to 4, **characterized in that** a head part (25) is configured on the fold-out part (7), wherein an actuation point (27) that is preferably connected to the linkage (8) is configured on the head part (25), in particular wherein the actuation point (27) is connected to a linkage arm (22, 26) of the or a toggle lever (18).

6. Instrument (1) according to one of Claims 1 to 5, **characterized in that** a connection of the actuation point (27) to the linkage (8) is configured such that the or a head part (25) of the fold-out part (7) in the first position of the fold-out part (7) and in the second position of the fold-out part (7) assumes alignments that are mutually offset, in particular mutually opposite.

7. Instrument (1) according to one of Claims 1 to 6, **characterized in that** the linkage (8) has a further adjustment device (28) by way of which at least the or a head part (25) of the fold-out part (7), at least in the second position, is adjustable relative to the main part (6) in the case of a stationary spindle drive (9).

8. Instrument (1) according to one of Claims 1 to 7, **characterized in that** the main part (6) and/or the fold-out part (7) are/is configured as a rigid part.

9. Instrument (1) according to one of Claims 1 to 8, **characterized in that** the fold-out part (7) in the first position is received in a receptacle (46) of the main part (6), and/or **in that** at least one operation and/or observation unit (29), in particular an illumination unit, a camera (30) and/or a laser light exit location (31) are/is configured on the main part (6) in a region on which the fold-out part (7) in the first position bears on the main part (6).

10. Instrument (1) according to one of Claims 1 to 9, **characterized in that** a feed installation (32) for a consumable material (33), in particular for a gas, a fluid, and/or a solid material, that is conveyed in the or an operation duct (17), is configured, and/or **in that** a machining unit, in particular a milling, boring and/or cutting unit, is configured on the main part (6) and/or on the fold-out part (7).

11. Instrument (1) according to one of Claims 1 to 10, **characterized in that** a laser fiber (38) and/or a laser light exit location (31) are/is fed in the stem (2) on the main part (6) and/or on the fold-out part (7), and/or in that the or a camera (30) and/or the or an illumination unit are/is configured on the fold-out part (7) and/or on the main part (6).

12. Instrument (1) according to one of Claims 1 to 11, **characterized in that** the instrument (1) is configured as a visualizing instrument, in particular as an endoscope.

13. Use of an instrument (1) according to one of the preceding claims for machining, in particular repairing, a component (41) that is disposed within a cavity (40), in particular an internal wall of a pipe or a component that is movable and/or exposed to a particulate flow, wherein the or a consumable material (33), in particular the or a gas, fluid and/or solid material, is conveyed by way of the instrument (1) into the cavity (40), by way of which consumable material (33) a clearance (43) at a defective location (42) of the component (41) is filled, and/or a material build-up is formed, and/or a coating and/or a surface finish is applied, and/or a joining method is carried out.

## Revendications

1. Instrument (1) comprenant une tige flexible (2), qui présente une extrémité distale (3) et une extrémité proximale (4), dans lequel une poignée (5) est disposée au niveau de l'extrémité proximale (4) et une partie principale (6) est formée au niveau de l'extrémité distale (3), dans lequel une partie rabattable (7) est disposée d'une façon déplaçable à l'aide d'une tringle (8) entre une première position disposée sur la partie principale (6) et une deuxième position espacée latéralement de la partie principale (6) par rapport à une direction d'étendue de la tige (2), dans lequel est formé un entraînement à broche (9), avec lequel la tringle (8) est actionnable, dans lequel une partie filetée (10) de l'entraînement à broche (9) peut être entraîné par l'intermédiaire d'un arbre flexible (12) qui est mené le long de la tige (2), et dans lequel l'entraînement à broche (9) comporte une partie à filet inverse (13), qui est reliée à la tringle (8) et qui contient la partie filetée (10) pour la formation de l'entraînement à broche (9), **caractérisé en ce que** la tringle (8) forme un levier articulé (18), dans lequel l'entraînement à broche (9) est attaché à une articulation (19) du levier articulé (18).

2. Instrument (1) selon la revendication 1, **caractérisé en ce que** l'arbre flexible (12) est disposé dans un canal de guidage (16) dans la tige (2), et/ou **en ce qu'**un canal de travail (17) qui se termine au niveau de la partie rabattable (7) est formé le long de la tige (2), en particulier dans lequel le canal de travail (17) est réalisé de façon flexible, en particulier de façon résistante à la traction et à la compression, dans une partie (47) qui est située entre la tige (2) et la partie rabattable (7).

3. Instrument (1) selon la revendication 1 ou 2, **caractérisé en ce qu'**une extrémité libre (20, 21) du levier articulé (18) est attachée à, en particulier articulée sur la partie principale (6) et/ou sur la partie rabattable (7).

4. Instrument (1) selon l'une quelconque des revendications 1 à 3, **caractérisé en ce qu'**un bras de tringle (22, 26) de la tringle (8) est attaché à, en particulier articulé par une première extrémité (20) sur la partie rabattable, et est guidé de façon coulissante, en particulier supporté latéralement, avec une deuxième extrémité (23) sur la partie principale (6).

5. Instrument (1) selon l'une quelconque des revendications 1 à 4, **caractérisé en ce qu'**une partie de tête (25) est formée sur la partie rabattable (7), dans lequel un point de commande (27) qui est relié de préférence à la tringle (8) est formé sur la partie de tête (25), en particulier dans lequel le point de commande (27) est relié à un bras de tringle (22, 26) du ou d'un levier articulé (18).

6. Instrument (1) selon l'une quelconque des revendications 1 à 5, **caractérisé en ce qu'**une liaison du point de commande (27) avec la tringle (8) est formée de telle manière que ladite ou une partie de tête (25) de la partie rabattable (7) présente, dans la première position de la partie rabattable (7) et dans la deuxième position de la partie rabattable (7), des orientations mutuellement décalées, en particulier opposées.

7. Instrument (1) selon l'une quelconque des revendications 1 à 6, **caractérisé en ce que** la tringle (8) comprend un autre dispositif de réglage (28), avec lequel au moins ladite ou une partie de tête (25) de la partie rabattable (7) est réglable, lorsque l'entraînement à broche (9) est bloqué au moins dans la deuxième position par rapport à la partie principale (6).

8. Instrument (1) selon l'une quelconque des revendications 1 à 7, **caractérisé en ce que** la partie principale (6) et/ou la partie rabattable (7) est (sont) réalisée(s) sous la forme d'une partie rigide.

9. Instrument (1) selon l'une quelconque des revendications 1 à 8, **caractérisé en ce que** la partie rabattable (7) dans la première position est logée dans un logement (46) de la partie principale (6) et/ou **en ce qu'**au moins une unité de travail et/ou d'observation (29), en particulier une unité d'éclairage, une caméra (30) et/ou un point de sortie de lumière laser (31), est formée sur la partie principale (6) dans une zone sur laquelle la partie rabattable (7) s'applique sur la partie principale (6) dans la première position.

10. Instrument (1) selon l'une quelconque des revendications 1 à 9, **caractérisé en ce qu'**un dispositif d'alimentation (32) est réalisé pour un matériau utilisé (33) qui est transporté dans un canal de travail (17), en particulier un gaz, un fluide ou un matériau solide, et/ou **en ce qu'**une unité de traitement, en particulier une unité de fraisage, de forage et/ou de coupe est formée sur la partie principale (6) et/ou sur la partie rabattable (7).

11. Instrument (1) selon l'une quelconque des revendications 1 à 10, **caractérisé en ce qu'**une fibre laser (38) est introduite dans la tige (2) et/ou est prévue au niveau d'un point de sortie de lumière laser (31) sur la partie principale (6) et/ou sur la partie rabattable (7), et/ou **en ce que** ladite ou une caméra (30) et/ou ladite ou une unité d'éclairage est (sont) formée(s) sur la partie rabattable (7) et/ou sur la partie principale (6).

12. Instrument (1) selon l'une quelconque des revendications 1 à 11, **caractérisé en ce que** l'instrument (1) est réalisé sous la forme d'un instrument de visualisation, en particulier d'un endoscope.

13. Utilisation d'un instrument (1) selon l'une quelconque des revendications précédentes pour un usinage, en particulier une réparation, d'un composant (41) qui est disposé à l'intérieur d'une cavité (40), en particulier d'une paroi intérieure de tube ou un composant mobile et/ou un composant exposé à un flux de particules, dans lequel ledit ou un matériau utilisé (33), en particulier ledit ou un gaz, fluide et/ou matériau solide, est transporté dans la cavité (40) à l'aide de l'instrument (1), matériau avec lequel on comble un évidement (43) en un endroit défectueux (42) du composant (41) et/ou on forme un amas de matériau et/ou on dépose un revêtement et/ou un améliorateur de surface et/ou on effectue une opération de jonction.
